# EUROPÄISCHE PATENTSCHRIFT

(11) **EP 2 121 102 B1**
(45) Veröffentlichungstag und Bekanntmachung des Hinweises auf die Patenterteilung: **27.03.2013**
(21) Anmeldenummer: 07856644.5
(22) Anmeldetag: 12.12.2007
(51) Int. Cl.: A61M 27/00

(54) **SAUGDRAINAGE MIT EINEM ANTIBAKTERIELL WIRKENDEN MATERIALDEPOT**
ASPIRATION DRAINAGE SYSTEM COMPRISING A MATERIAL DEPOT WITH AN ANTI-BACTERIAL ACTION
SYSTÈME DE DRAINAGE D'ASPIRATION AVEC DÉPÔT DE MATIÈRE À ACTION ANTIBACTÉRIENNE

(30) Priorität: 20.12.2006 DE 102006060934
(43) Veröffentlichungstag der Anmeldung: 25.11.2009
(73) Patentinhaber: Wolter, Dietmar, 22955 Hoisdorf (DE)
(72) Erfinder: Wolter, Dietmar, 22955 Hoisdorf (DE)
(74) Vertreter: Glawe, Delfs, Moll
(86) Internationale Anmeldenummer: PCT/EP2007/010897
(87) Internationale Veröffentlichungsnummer: WO 2008/077492

(56) Entgegenhaltungen:
- EP-A- 0 207 250
- WO-A-03/028786
- DE-A1- 3 115 763
- DE-A1- 3 441 586

## Beschreibung

Die Erfindung betrifft eine Saugdrainage zum Absaugen von Sekreten und anderen Flüssigkeiten aus dem menschlichen oder tierischen Körper, die mit einem antibakteriell wirkenden Materialdepot versehen ist.

Es ist bekannt, dass durch die Anwendung von Saugdrainagen, z.B. Redon-Saugdrainagen die Blutergussbildung und Gewebswasseransammlungen in Operationswunden wesentlich vermindert werden kann. Dies führt zu einer besseren Wundheilung, da die Gewebsschichten sich aneinanderlegen können und dadurch direkt verheilen.

Bei jedem operativen Eingriff kommt es zur Kontamination der Wunde mit Keimen. Bei einer Saugdrainage, welche über eine zusätzliche Hautöffnung außerhalb der eigentlichen Wunde nach außen geführt wird, lässt sich beobachten, dass auch ein Eindringen von Keimen von außen über die Drainage, das Lumen oder die Außenseite selbst stattfindet.

Dieser Vorgang der Keiminvasion bei liegender Drainage ereignet sich also nicht nur an der Außenseite der Drainage sondern auch bei unsachgemäßem Gebrauch, insbesondere bei Wechsel einzelner Systemkomponenten über das Drainagelumen. Trägt der behandelnde Arzt oder die Pflegekraft z.B. keine sterilen Handschuhe und führt nicht in ausreichendem Maße eine Desinfektion durch, so kann es beim Wechsel einzelner Systemkomponenten, z.B. beim Wechsel der Vakuumflasche, zur Kontaminierung des Drainagelumens und seines Inhaltes mit pathologischen Keimen kommen, welche das gesamte System und die Wunde besiedeln.

Aus EP 0 476 503 ist bekannt, dass den Keimen durch ein antibakteriell wirkendes Materialdepot, beispielsweise ein mit Gentamycin beladener Polymethylmethacrylat (PMMA)-Pfropfen am Ende des in den Körper einzubringenden Saugschlauches der Saugdrainage, entgegengewirkt werden kann. Das aus dem Materialdepot lokal abgegebene antibakterielle Material wirkt nicht nur gegenüber Keimen, die über die Außenseite der Drainage oder über das Lumen in die Wunde gelangt sind, sondern auch gegenüber Keimen, die bei der Operation in die Wunde gelangt sind.

Das antibakteriell wirkende Material tritt in der Wunde aus dem Materialdepot aus und gelangt mit den Sekreten und anderen Flüssigkeiten in der Wunde und vermischt sich dort mit der Wundflüssigkeit. Gleichzeitig gelangt das Wundsekret zu den Öffnungen des Saugschlauches durch die es abgesaugt wird. Verglichen mit einer systemischen Antibiotika-Therapie lassen sich auf diese Art am Wundort höhere Wirkstoffkonzentrationen erreichen, ohne dass der Gesamtkörper mit kurativen Antibiotika-Mengen belastet wird.

Es ist bekannt, dass die Abtötung der Keime nicht allein von der Anwesenheit des Antibiotikums in dem das Bakterium umgebenden Milieu, sondern auch der Konzentration des Antibiotikums (Antibiotika-Spiegel) im Gewebe abhängig ist. Die lokale Behandlung der Wunde mit einer Saugdrainage mit einem antibakteriell wirkenden Materialdepot ist vielfach noch wirksam, wenn in mikrobiologischen Testungen eine "einfache" Antibiotika-Resistenz nachgewiesen wird, da in den bakteriologischen Testungen zumeist nur solche Antibiotika-Konzentrationen geprüft werden, wie sie im Zuge einer intravenösen Therapie erreichbar sind.

Es ist bekannt, dass auch bei der Verwendung von Saugdrainagen mit einem antibakteriell wirkenden Materialdepot der eingangs genannten Art der Fall eintreten kann, dass zu wenig Antibiotikum abgegeben wird. Das von dem Materialdepot abgegebene antibakterielle Material (Antibiotikum) gelangt bei stärkerer Blutung, und damit höherer Absaugemenge, zu schnell zu den Öffnungen des Saugschlauches, durch die es abgesaugt wird, noch bevor sich der erwünschte Antibiotika-Spiegel in der Wunde eingestellt hat.

WO 03/028786 A2 und EP 0 207 250 A1 offenbaren Absaugeinrichtungen mit Medikamentendepots.

Die Aufgabe der Erfindung besteht in der Schaffung einer Saugdrainage der eingangs genannten Art, die die vorgenannten Nachteile nicht aufweist.

Die Erfindung löst diese Aufgabe durch eine Saugdrainage mit den Merkmalen des Anspruchs 1. Vorteilhafte Ausführungsformen der Erfindung sind in den Unteransprüchen offenbart.

Gemäß Anspruch 1 ist die Oberfläche des am Ende des in den Körper einzubringenden Saugschlauches der Saugdrainage angeordneten Materialdepots, über die das antibakterielle Wirkstoffmaterial in den menschlichen oder tierischen Körper abgegeben wird, größer ist als die Einhüllende des Materialdepots.

Die Einhüllende (auch Envelope) bezeichnet eine um einen Körper gespannte geschlossene Fläche. In Querschnittsdarstellung gilt, dass die Einhüllende jede Kurve einer Kurvenschar in einem Punkt berührt. Beispielsweise wird die Einhüllende einer Kugel mit einem Grundradius r und sich in den Innenraum der Kugel erstreckenden Rillen bestimmt durch die Kugeloberfläche, wie sie sich aus dem Grundradius ergibt (4πr²).

Die Erfindung hat erkannt, dass die Menge eines antibakteriellen Wirkstoffmaterials, das über die Oberfläche des Materialdepots abgegeben wird, von dem Gehalt des Wirkstoffmaterials im Träger bzw. der Beladung des Trägermaterials mit dem Wirkstoffmaterial abhängig ist und diesem Gehalt bzw. der Beladung durch das Trägermaterial vorgegebene Obergrenzen gesetzt sind. Die Erfindung hat ebenfalls erkannt, dass das Antibiotikum aus dem Trägermaterial nur aus der obersten Schicht des Trägermaterials herausgelöst wird. Bei Untersuchungen von explantierten Gentamycin-PMMA-Ketten, die auch unter dem Begriff der Septopal^{®}-Ketten dem Fachmann geläufig sind, aber auch aus explantiertem Gentamycin-haltigen Knochenzementmaterial bei Prothesenwechsel, konnte nachgewiesen werden, dass das antibiotische Material Gentamycin lediglich aus dem wenige Millimeter dicken äußeren Bereich herausgelöst wird, während die tieferen Schichten gleich bleibende Antibiotika-Anteile aufweisen. Die Erfindung hat des Weiteren erkannt, dass durch eine Vergrößerung der Oberfläche des Materialdepots mehr Antibiotikum pro Zeiteinheit abgegeben werden kann, d.h. ein ausreichend hoher Antibiotika-Spiegel eingestellt werden kann, selbst wenn es zu einem schnellen Abtransport des Antibiotikums mit dem Wundsekret und anderen Flüssigkeiten durch die Öffnungen des Saugschlauches kommt. Einer Antibiotika-Unterdosierung kann dadurch entgegengewirkt werden. Die hohe lokale Antibiotikakonzentration führt zu effektiver Abtötung nicht nur empfindlicher pathologischer Keime, sondern auch partiell resistenter Keime.

Die Erfindung hat ebenfalls erkannt, dass das erfindungsgemäße Materialdepot nicht zu einer ebenfalls als problematisch angesehenen Antibiotika-Überdosierung führt. Es hat sich gezeigt, dass das aus dem Materialdepot in die Wundflüssigkeit abgegebene Antibiotikum in der Wundflüssigkeit durch die Dynamik der Wundflüssigkeit, z.B. durch Lagewechsel des Patienten oder durch Muskeltätigkeit, verteilt wird und sich ein für den Patienten ungefährlicher Antibiotika-Spiegel lokal, aber auch im Blutspiegel allgemein, einstellt. Sofern im Einzelfall eine Überdosierung nicht ausgeschlossen werden kann oder eine verzögerte Freisetzung des Antibiotikums erwünscht ist, ist es zweckmäßig, die Geschwindigkeit der Antibiotika-Mengenabgabe entsprechend einzustellen. Dies kann durch geeignete Mengenverhältnisse des Antibiotikums im Materialdepot und/oder im Materialdepot eingebaute Zusatzstoffe erfolgen, die die Freisetzung des Antibiotikums verzögern. Die Geschwindigkeit der Freisetzung kann auch dadurch gesteuert werden, indem das Materialdepot unterschiedlich stark gepresst wird oder z.B. schaumförmig aufgebaut wird.

Die Vergrößerung der Oberfläche des Materialdepots kann durch verschiedenste Oberflächenstrukturen, beispielsweise Vertiefungen, muldenförmigen Ausnehmungen, Einstülpungen und Ausstülpungen wie Furchen, Falten, Schlitze, Lamellen, Waben und Bohrungen erfolgen. Die Furchen, Falten und Schlitze sind längs, quer oder gewindeartig, vorzugsweise längs der Saugdrainageschlauchachse oder gewindeartig um die Saugdrainageschlauchachse, in der Oberfläche des Materialdepots angelegt. Vorteilhafterweise beschädigen, reizen oder beeinträchtigen die Oberflächenstrukturen das Gewebe vor Ort nicht. So weist das Materialdepot vorteilhafterweise keine scharfen Kanten auf, so dass beim Entfernen der Saugdrainage eine Verletzung des Gewebes, der Nerven und Gefäße und ein Nachbluten weitestgehend vermieden werden kann. Die erfindungsgemäße Anordnung der Furchen, Falten und Schlitze längs der Saugdrainageschlauchachse unterstützt das ungehinderte Herausgleiten der Saugdrainage beim Herausziehen aus der Wunde.

Die Tiefen oder Höhen der Oberflächenstrukturen sind zweckmäßigerweise so gewählt, dass einerseits die Festigkeit des Trägermaterials nicht beeinträchtigt ist und beispielsweise ein unerwünschter Abbruch von Trägermaterial beim Einbringen oder Entfernen des Saugschlauches mit dem endständig angeordneten Materialdepot vermieden wird, andererseits die Materialdicke zwischen den benachbarten Strukturen des Materialdepots so stark ist, dass eine möglichst effektive Wirkstoffabgabe aus dem Trägermaterial in der Wunde erfolgt. So verfügen z.B. Lamellen, d.h. lange Längsrillen, besonders wenn sie zu mehreren mit ihren Flächen parallel oder annähernd parallel zueinander angeordnet und miteinander verbunden sind, über eine am Materialaufwand gemessen sehr große Oberfläche und Stabilität. Berücksichtigt man, dass das antibiotische Material Gentamycin aus dem Trägermaterial PMMA lediglich aus einem wenige Millimeter dicken Hüllenbereich in der Wunde herausgelöst wird, sollte die Materialdicke zwischen den benachbarten Strukturen des Materialdepots vorzugsweise nur wenige Millimeter, vorzugsweise höchstens 5 mm, weiter vorzugsweise 3 mm, besonders bevorzugt 1 bis 3 mm betragen.

Die Strukturierung des erfindungsgemäßen Materialdepots erlaubt es, die Oberfläche des Materialdepots gegenüber der Einhüllenden mindestens um den Faktor 1,1, 1,2, 1,3, 1,4, 1,5, 1,6, 1,7, 1,8, 1,9 oder um ein Vielfaches (mindestens um den Faktor 2, 3, 4, 5, 6, 7, 8, 9, 10, 11-100, 100-200, 200-300, 300-400, 400-500, 500-600, 600-700, 700-800, 800-900 oder 900-1000) zu erhöhen.

Zweckmäßigerweise ist das antibakteriell wirkende Material ein Antibiotikum, wobei besonders zweckmäßig Gentamycin ist. Alternativ zu oder in Kombination mit Gentamycin können auch weitere Antibiotika, beispielsweise Vanvomycin und/oder Clindamycin verwendet werden.

Das antibakteriell wirkende Material kann in einem resorbierbaren Trägermaterial (z.B. Polyglycolid-Lactid oder Collagen) oder in einem nicht resorbierbaren Material (z.B. PMMA) eingeschlossen sein. Vorzugsweise ist das Materialdepot an seiner Oberfläche, zumindest teilweise, offenporig. Die offenen Poren erlauben eine erleichterte Abgabe des antibakteriellen Wirkstoffmaterials in der Wunde. Gemäß einer bevorzugten Ausführungsform der Erfindung ist das resorbierbare Materialdepot lösbar am Ende des in den Körper einzubringenden Saugschlauches angeordnet, beispielsweise mittels einer Sollbruchstelle zwischen Saugschlauch und Materialdepot. Wenn die Absaugung abgeschlossen ist, kann das Materialdepot weiterhin noch keimtötend wirken.

Die durch die Einhüllende gebildete geometrische Grundform des Materialdepots ist vorzugsweise eine Form, ausgewählt aus der Gruppe bestehend aus Zylinder, Kugel, Kegel sowie der Kombination dieser Grundformen wie der Tropfenform. Grundsätzlich ist jede Grundform bevorzugt, die es erlaubt, das Ende des Saugschlauches mit dem Materialdepot für den Patienten schonend in den Körper einzubringen und/oder zu entfernen. "Schonend" bedeutet insbesondere, dass die Wundöffnung zum Einbringen und/oder Entfernen des Saugschlauches mit dem Materialdepot möglichst klein gehalten werden kann, jedoch eine leichte Entfernung trotzdem erlaubt.

Die erfindungsgemäße Saugdrainage kann weiterhin eine Kette von Materialdepots der erfindungsgemäßen Art am Ende des in den Körper einzubringenden Saugschlauches aufweisen. Mit anderen Worten, das am Ende des in den Körper einzubringenden Saugschlauches angeordnete Materialdepot ist um mindestens ein weiteres Materialdepot kettengliedartig verlängert. Die Kettenglieder können auf einem zentralen Draht angeordnet sein. Die Kettenglieder können z.B. eine sphärische Grundform, analog der aus dem Stand der Technik bekannten Septopal^{®}-Ketten, oder eine Walzenform, beispielsweise eine Vollzylinderform, aufweisen. Die benachbarten Kettenglieder können auch zueinander korrespondierende Flächen aufweisen. Die Flächen können z.B. konvex-konkav ausgebildet sein. Dadurch ist auch bei einem geringen Abstand zwischen den benachbarten Kettengliedern eine gelenkartige Bewegung der Kettenglieder möglich, die es erlaubt die Saugdrainage leichter in den Körper einzubringen, bei Bedarf zu (re-)positionieren oder zu entfernen. Derartige Kettenformen haben den Vorteil, dass sie flexibel-sind und sich den lokalen Gegebenheiten anpassen lassen.

An dieser Stelle sei noch einmal darauf hingewiesen, dass die vorgenannten Vorteile der Saugdrainagen nicht nur bei Saugdrainagen gegeben sind, die in Operationswunden eingelegt werden. Ähnliche Vorteile treten vielmehr auch in anderen Fällen auf, bei denen ein Sekret aus dem menschlichen oder tierischen Körper abgesaugt werden muss. Als Beispiele seien hier noch die Thorax-Drainage oder ein Blasenkatheter genannt.

Die Erfindung wird im Folgenden anhand von vorteilhaften Ausführungsformen unter Bezugnahme auf die beigefügten Zeichnungen beschrieben. Es zeigen:
- Fig. 1: eine erste Ausführungsform eines Saugschlauches mit einem Materialdepot, das für die Saugdrainage der Erfindung verwendet werden kann;
- Fig. 2: eine zweite Ausführungsform;
- Fig. 3: eine dritte Ausführungsform; und
- Fig. 4: eine vierte Ausführungsform.

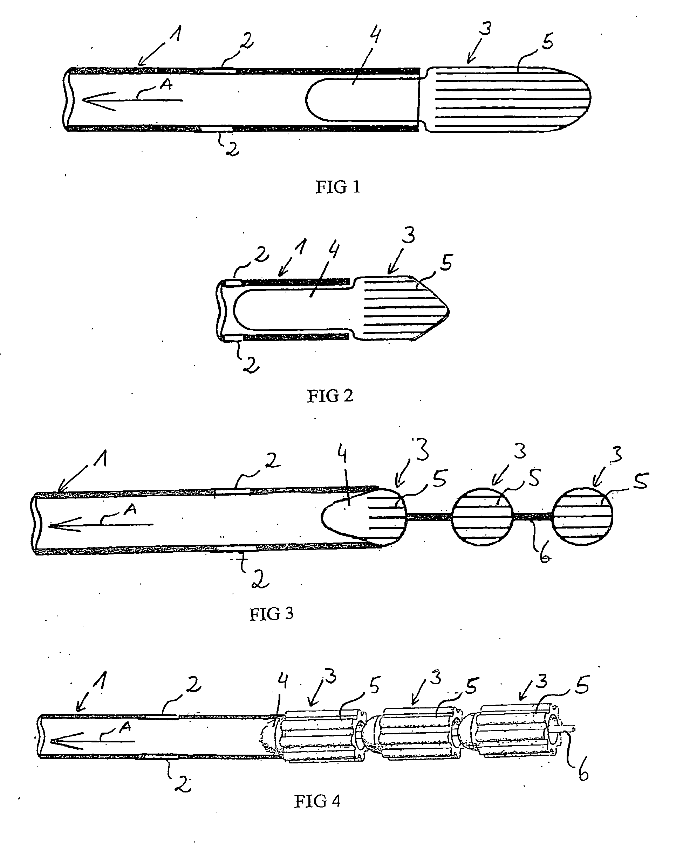

In Figur 1 ist ein Saugschlauch 1 im Querschnitt gezeigt, der mit Öffnungen 2 versehen ist, durch die das Sekret oder andere Körperflüssigkeiten abgesaugt werden kann. Der Saugschlauch 1 schließt an seinem in den Körper einzubringenden Ende mit einem in der Seitenansicht dargestellten Materialdepot 3 ab. Der Saugschlauch 1 ist an eine Unterdruckstelle (nicht dargestellt) angeschlossen, so dass das Sekret in Richtung des Pfeils A abgesaugt wird. Der Saugschlauch 1 ist an seinem im Körper angeordneten Ende mit dem Schaft 4 des Materialdepots 3 verpfropft. Der sich aus dem Schaft 4 erstreckende Bereich des Materialdepots 3 ist walzenförmig. Die Oberfläche des Materialdepots 3 ist durch längs der Saugschlauchachse angeordnete Rillen 5 vergrößert. Das Materialdepot 3 enthält das antibakterielle Wirkstoffmaterial (nicht dargestellt). Das antibakteriell wirkende Material verlässt das Materialdepot 3 bis es durch die Öffnungen 2 eingesaugt wird.

Die in Figur 2 dargestellte Ausführungsform entspricht der in Figur 1 Dargestellten, mit dem Unterschied, dass der sich aus dem Schaft 4 des Materialdepots 3 erstreckende Bereich tropfenförmig ist. Die tropfenförmige Verjüngung des Materialdepots 3 ermöglicht eine leichtere Entfernung der Saugdrainage.

Die in den Figur 3 dargestellte Ausführungsform entspricht der in Figur 1 Dargestellten, mit dem Unterschied, dass das am Saugschlauch 1 verpfropfte Materialdepot 3 um zwei weitere Materialdepots 3 in Kugelform verlängert wurde, die auf einem zentralen Draht 6 fixiert sind.

Die in Figur 4 dargestellte Ausführungsform ähnelt der in Figur 3 Dargestellten. Eine Kette aus walzenförmigen Materialdepots 3 ist auf einem zentralen Draht 6 fixiert und ein endständiges Materialdepotkettenglied 3 ist mit dem Saugschlauch 1 verpfropft. Die walzenförmigen Materialdepots 3 weisen an Stelle der bei einem Zylinder üblichen gegenüberliegenden planen Flächen einander korrespondierende konkav-konvexe Flächen auf. Dadurch ist auch bei einem geringen Abstand zwischen den benachbarten Kettengliedern 3 eine gelenkartige Bewegung der Kettenglieder möglich.

## Patentansprüche

1. Saugdrainage zum Absaugen von Sekreten und anderen Flüssigkeiten aus dem menschlichen oder tierischen Körper mit einem Saugschlauch (1), der mit Öffnungen (2) versehen ist, und mindestens einem am Ende des in den Körper einzubringenden Saugschlauches (1) angeordneten Materialdepot (3) zur Abgabe eines antibakteriellen Wirkstoffmaterials, wobei die Oberfläche des Materialdepots (3) größer ist als die Einhüllende des Materialdepots,
**dadurch gekennzeichnet*,* dass** der Saugschlauch (1) an dem Ende mit dem Materialdepot (3) verpfropft ist.

2. Saugdrainage nach Anspruch 1, **dadurch gekennzeichnet, dass** die Oberfläche des Materialdepots (3) mindestens um den Faktor 1,1, vorzugsweise mindestens um den Faktor 2, besonders bevorzugt mindestens um den Faktor 3 größer ist als die Einhüllende des Materialdepots (3).

3. Saugdrainage nach Anspruch 1 oder 2, **dadurch gekennzeichnet, dass** das Materialdepot (3) mindestens eine Oberflächenstruktur (5) aufweist, ausgewählt aus der Gruppe bestehend aus: Vertiefungen, muldenförmigen Ausnehmungen, Einstülpungen und Ausstülpungen wie Furchen, Falten, Schlitze, Lamellen, Waben und Bohrungen.

4. Saugdrainage nach Anspruch 3, **dadurch gekennzeichnet, dass** die Furchen, Falten und Schlitze (5) längs, quer oder gewindeartig, vorzugsweise längs der Saugdrainageschlauchachse oder gewindeartig um die Saugdrainageschlauchachse, in der Oberfläche des Materialdepots (3) angelegt sind.

5. Saugdrainage nach einem der vorangehenden Ansprüche, **dadurch gekennzeichnet, dass** das antibakterielle Wirkstoffmaterial in einem vom Körper zu resorbierenden Trägermaterial und/oder einem vom Körper nicht zu resorbierenden Trägermaterial eingeschlossen ist.

6. Saugdrainage nach einem der Ansprüche 3 bis 5, **dadurch gekennzeichnet, dass** die Materialdicke zwischen den benachbarten Strukturen (5) des Materialdepots (3) 5 mm, vorzugsweise 3 mm, besonders bevorzugt 1 bis 3 mm nicht überschreitet.

7. Saugdrainage nach einem der vorangehenden Ansprüche, **dadurch gekennzeichnet, dass** das Materialdepot (3) an seiner Oberfläche, zumindest teilweise, offenporig ist.

8. Saugdrainage nach einem der vorangehenden Ansprüche, **dadurch gekennzeichnet, dass** die geometrische Grundform des Materialdepots (3) ausgewählt ist aus der Gruppe bestehend aus Zylinder, Kugel, Kegel sowie der Kombination dieser Grundformen wie der Tropfenform.

9. Saugdrainage nach einem der vorangehenden Ansprüche, **dadurch gekennzeichnet, dass** das Materialdepot (3) lösbar am Ende des in den Körper einzubringenden Saugschlauches (1) angeordnet ist.

10. Saugdrainage nach einem der vorangehenden Ansprüche, **dadurch gekennzeichnet, dass** sie mindestens zwei Materialdepots (3) gemäß den vorangehenden Ansprüchen als Glieder einer Kette aufweist.

11. Saugdrainage nach Anspruch 10, **dadurch gekennzeichnet, dass** die gegenüberstehenden Flächen der benachbarten Materialdepotkettenglieder (3) der Form nach, zumindest teilweise, korrespondieren.

12. Saugdrainage nach Anspruch 11, **dadurch gekennzeichnet, dass** die gegenüberstehenden Flächen der benachbarten Materialdepotkettenglieder (3) konkavkonvex ausgebildet sind.

## Claims

1. An aspiration drainage system for aspirating secretions and other fluids out of the human or animal body using a suction tube (1), which is provided with openings (2), and at least one material depot (3), arranged at the end of the suction tube (1) to be inserted into the body, for dispensing an antibacterial active ingredient material, the surface of the material depot (3) being larger than the envelope of the material depot, **characterized in that** the suction tube (1) is plugged at the end with the material depot (3).

2. The aspiration drainage system as claimed in claim 1, **characterized in that** the surface of the material depot (3) is larger than the envelope of the material depot (3) by at least a factor of 1.1, preferably at least a factor of 2, particularly preferably at least a factor of 3.

3. The aspiration drainage system as claimed in claim 1 or 2, **characterized in that** the material depot (3) has at least one surface structure (5) selected from the group composed of: depressions, hollow recesses, indentations and protuberances such as channels, folds, slits, lamellae, combs and bores.

4. The aspiration drainage system as claimed in claim 3, **characterized in that** the channels, folds and slits (5) are established along, across or, in a thread-like manner, in the surface of the material depot (3), preferably along the aspiration drainage tube axis or in a thread-like manner about the aspiration drainage tube axis.

5. The aspiration drainage system as claimed in one of the preceding claims, **characterized in that** the antibacterial active ingredient material is enclosed in a carrier material which is intended to be reabsorbed by the body and/or a carrier material which is not intended to be reabsorbed by the body.

6. The aspiration drainage system as claimed in one of claims 3 to 5, **characterized in that** the material thickness between the adjacent structures (5) of the material depot (3) does not exceed 5 mm, preferably 3 mm, particularly preferably 1 to 3 mm.

7. The aspiration drainage system as claimed in one of the preceding claims, **characterized in that** the material depot (3) has, at least in parts, open pores on its surface.

8. The aspiration drainage system as claimed in one of the preceding claims, **characterized in that** the basic geometric shape of the material depot (3) is selected from the group composed of cylinder, sphere, cone and the combination of these basic shapes such as the teardrop shape.

9. The aspiration drainage system as claimed in one of the preceding claims, **characterized in that** the material depot (3) is arranged detachably at the end of the suction tube (1) to be inserted into the body.

10. The aspiration drainage system as claimed in one of the preceding claims, **characterized in that** it has at least two material depots (3) according to the preceding claims as elements in a chain.

11. The aspiration drainage system as claimed in claim 10, **characterized in that** the opposing faces of the adjacent material depot chain elements (3) have a corresponding shape, at least in part.

12. The aspiration drainage system as claimed in claim 11, **characterized in that** the opposing faces of the adjacent material depot chain elements (3) have a concave-convex design.

## Revendications

1. Système de drainage par aspiration permettant d'aspirer les sécrétions et autres liquides émanant du corps humain ou animal, comportant un tuyau flexible d'aspiration (1) muni d'ouvertures (2), et au moins un dépôt de matières (3), agencé à l'extrémité du tuyau flexible d'aspiration (1) à introduire dans le corps et fournissant une matière à action antibactérienne, la surface du dépôt de matières (3) étant supérieure à l'enveloppe du dépôt de matières (3), **caractérisé en ce que** le tuyau flexible d'aspiration (1) est bouché à l'extrémité comprenant le dépôt de matières (3).

2. Système de drainage par aspiration selon la revendication 1, **caractérisé en ce que** la surface du dépôt de matières (3) est au moins 1,1 fois, de préférence au moins 2 fois, encore mieux au moins 3 fois supérieure à l'enveloppe du dépôt de matières (3).

3. Système de drainage par aspiration selon la revendication 1 ou 2, **caractérisé en ce que** le dépôt de matières (3) comporte au moins une structure superficielle (5), choisie dans le groupe contenant des creux, des évidements en forme de cuvettes, des emboutissages en creux et en relief, tels que des rainures, des plis, des fentes, des lamelles, des alvéoles et des forures.

4. Système de drainage par aspiration selon la revendication 3, **caractérisé en ce que** les rainures, les plis et les fentes (5) sont réalisés longitudinalement, transversalement ou hélicoïdalement, de préférence le long de l'axe du tuyau flexible de drainage ou hélicoïdalement autour de l'axe du tuyau flexible de drainage, dans la surface du dépôt de matières (3).

5. Système de drainage par aspiration selon l'une quelconque des revendications précédentes, **caractérisé en ce que** la matière à action antibactérienne est enfermée dans un matériau support que le corps résorbe et/ou dans un matériau support que le corps ne résorbe pas.

6. Système de drainage par aspiration selon l'une quelconque des revendications 3 à 5, **caractérisé en ce que** l'épaisseur de matière entre les structures (5) adjacentes du dépôt de matières (3) ne dépasse pas 5 mm, de préférence 3 mm, encore mieux ne dépasse pas 1 à 3 mm.

7. Système de drainage par aspiration selon l'une quelconque des revendications précédentes, **caractérisé en ce que** la surface du dépôt de matières (3) est réalisée, au moins en partie, à pores ouverts.

8. Système de drainage par aspiration selon l'une quelconque des revendications précédentes, **caractérisé en ce que** la forme de base géométrique du dépôt de matières (3) est choisie dans un groupe contenant des cylindres, des sphères, des cônes, ainsi qu'une combinaison de ces formes de base, telle qu'une forme ovoïde.

9. Système de drainage par aspiration selon l'une quelconque des revendications précédentes, **caractérisé en ce que** le dépôt de matières (3) est disposé de manière amovible à l'extrémité du tuyau flexible d'aspiration (1) à introduire dans le corps.

10. Système de drainage par aspiration selon l'une quelconque des revendications précédentes, **caractérisé en ce que** ledit système comporte au moins deux dépôts de matières (3) selon les revendications précédentes, lesquels forment les maillons d'une chaîne.

11. Système de drainage par aspiration selon la revendication 10, **caractérisé en ce que** les surfaces opposées des maillons adjacents de la chaîne de dépôts de matières (3) ont, au moins en partie, des formes correspondantes.

12. Système de drainage par aspiration selon la revendication 11, **caractérisé en ce que** les surfaces opposées des maillons adjacents de la chaîne de dépôts de matières (3) sont réalisées sous forme concave-convexe.
